# EUROPEAN PATENT APPLICATION

(11) **EP 1 334 700 A1**
(43) Date of publication of application: **13.08.2003**
(21) Application number: 03250847.5
(22) Date of filing: 11.02.2003
(51) Int. Cl.: A61B 19/00

(54) **Active trocar**

(30) Priority: 12.02.2002 JP 2002034514
(71) Applicant: The University of Tokyo, Bunkyo-Ku, Tokyo (JP)
(72) Inventor: Nakamura, Yoshihiko, Tokyo (JP); Okada, Masafumi, Tokyo (JP); Kobayashi, Yuki, Tsukuba City, Ibaraki Pref. (JP)
(74) Representative: Fenlon, Christine Lesley

(57) **Abstract**

Provided is a small-sized surgery support robot (active trocar), in which the occupied range of space within the operating room is reduced with hardly reducing any degrees of freedom. The active trocar includes a first parallel crank quadric chain 7 containing a base link 3 supported on a base 2, which is fixed to a surgical bed, to be swingable around a first swinging axis O1; a second parallel crank quadric chain 11 containing two links 4 and 6 respectively unitized with links 4 and 6 of the first parallel crank quadric chain 7, in which a support link 10 of the four links intersecting the base link 3 is swung around a second swinging axis 02 intersecting the first swinging axis O1 at a right angle at a position spaced from the base link 3, and the support link supports the tube 13 in such an orientation that a central axis OT of the tube passes an intersection point P so as to be able to freely advance and retract the tube; a first swing drive means 16 for swinging the base link 3 around the first swinging axis O1 with respect to the base 2; a second swing drive means 17 for swinging the link 4 with respect to the base link 3; and an advancement and retraction drive means 18 for advancing and retracting the tube 13 with respect to the support link 10.

## Description

The present invention relates to an active trocar for swinging a tube inserted in a hole, which is opened in an abdominal wall of a body on a surgical bed, around a vicinity of a center of the hole and advancing/retracting the tube.

In laparoscopic surgery, a small hole is opened in an abdominal wall of a patient body, and an instrument such as a forceps and an endoscope is inserted in the hole. Then, an operation such as excision and suture is performed in an abdominal cavity. This is an operation with a large burden for a surgeon because of difficulty in operating the instrument. Therefore, in recent years, a lot of technological developments have been made in a robot supporting the laparoscopic surgery.

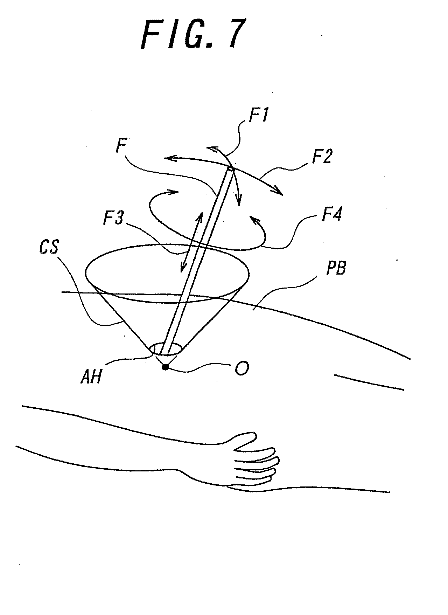

In performance of the operation in the abdominal cavity, in order to obtain sufficient degrees of freedom at a tip of the instrument such as the forceps, it is desirable to secure six degrees of freedom with regard to a position and an orientation of the instrument in the abdominal cavity by means of both of the instrument and a mechanism for moving the instrument. Fig. 7 shows a motion of a forceps. As shown in the drawing, a forceps F is moved within a conical space CS having a point O as a center in the vicinity of a center of a hole AH in an abdominal wall of a patient body PB. Accordingly, the mechanism driving the forceps F generally has four degrees of freedom F1 to F4 shown by arrows in Fig. 7.

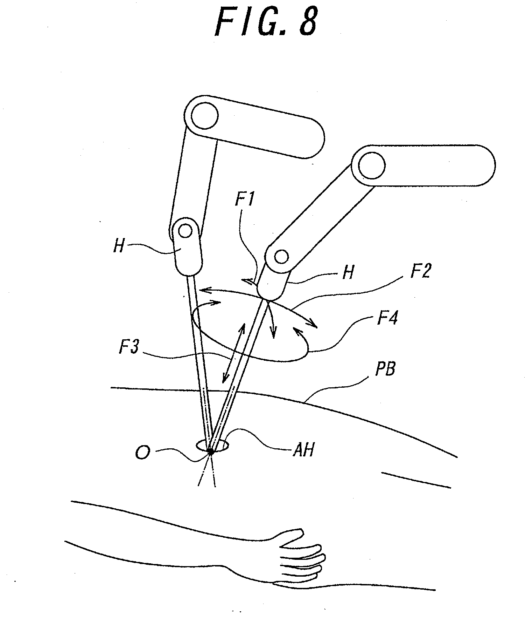

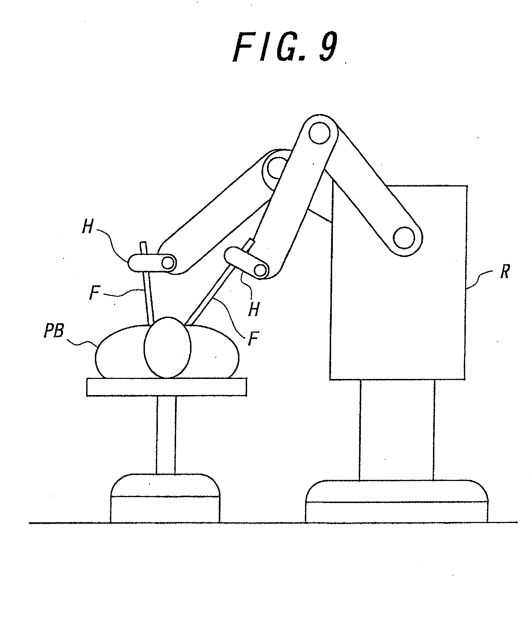

In a previously proposed method of driving a forceps, as shown in Fig. 8, an upper end of the forceps F is operated by a hand H of a robot such that the center.0 of swing (center of rotation) of the forceps F is located in the vicinity of the center of the hole AH in the abdominal wall. Such a robot needs to secure a wide operation range and high rigidity, as well as the robot realizes the same motion as human beings operate the forceps. Therefore, as shown in Fig. 9, the robot for driving a forceps was hitherto realized as a large-sized arm type (manipulator type) master-slave robot R.

However, the large-sized arm type robot R as described above occupies large space within an operating room, so that the robot prevents a doctor from accessing the patient at the time of emergency. Furthermore, because of a vicious circle of securement of rigidity of a long extending arm and an increase in power of an actuator moving the arm, the robot will increasingly become larger. Thus, downsizing of the robot driving an instrument such as the forceps while securing the rigidity is the largest requirement desired for the current surgery support robot.

The present invention seeks to provide an active trocar for swinging a tube inserted in a hole, which is opened in the abdominal wall of a body on a surgical bed, around a position of the hole as a center and advancing/retracting the tube, and for driving an instrument such as forceps inserted through the tube as a small-sized surgery support robot. In preferred embodiments of the present invention, the robot and the instrument such as the forceps are unitised and actuators therefore are concentratedly located near the abdominal wall. Moreoever, axes of three degrees of freedom, which are two axes of swing and an axis of linear movement, are allowed to intersect at one point in the vicinity of the center of the hole in the abdominal wall, so that the occupied range of space within the operating room is reduced while hardly reducing any degrees of freedom from that in the conventional one.

In order to alleviate the above mentioned problems, the active trocar embodying the present invention swings a tube inserted in a hole, which is opened in an abdominal wall of a body on a surgical bed, around a vicinity of a center of the hole and advances and retracts the tube. The active trocar includes a first parallel crank quadric chain, a second parallel crank quadric chain, a first swing drive means, a second swing drive means, and an advancement and retraction drive means. The first parallel crank quadric chain includes four links containing a base link, which is supported on a base to be swingable around a first swinging axis, the base being relatively fixed to the surgical bed. The four links are coupled to each other to be individually rotatable around rotating axes parallel to each other and orthogonal to the first swinging axis. Lines each connecting two rotating axes of the respective links form a parallelogram. The second parallel crank quadric chain includes four links containing two links respectively unitized with the link of said first parallel crank quadric chain rotatably coupled to the base link and the link in which a line connecting the two rotating axes is parallel to the line connecting the two rotating axes of the base link. The four links are coupled to each other to be individually rotatable around rotating axes parallel to each other and parallel to the rotating axis of said first parallel crank quadric chain. Lines each connecting two rotating axes of the respective links form a parallelogram. A support link among the remainder other than the foregoing two links in the four links, in which a line connecting the two rotating axes thereof intersects the line connecting the two rotating axes of the base link, is swung around a second swinging axis intersecting the first swinging axis at a right angle at a position spaced from the base link. Moreover, the support link supports the tube in such an orientation that a central axis of the tube passes an intersection point of the first swinging axis and the second swinging axis so as to be able to advance and retract the tube in an extending direction of the central axis of the tube. The first swing drive means swings the base link around the first swinging axis with respect to the base to swing the support link around the first swinging axis. The second swing drive means swings the link of the first parallel crank quadric chain rotatably coupled to the base link with respect to the base link to swing the support link around the second swinging axis. The advancement and retraction drive means advances and retracts the tube in the extending direction of the central axis of the tube with respect to the support link.

In the active trocar, the base is relatively fixed to the surgical bed such that an intersection point of the first swinging axis, the second swinging axis, and the central axis of the tube is located in the vicinity of the center of the hole opened in the abdominal wall of the body on the surgical bed. When an instrument such as the forceps is inserted through the tube, and then the first swing drive means, the second swing drive means, and the advancement and retraction drive means are properly operated, the first swing drive means swings, with respect to the base, the base link around the first swinging axis to swing the support link around the first swinging axis. Moreover, the second swing drive means swings, with respect to the base link, the link of the first parallel crank quadric chain rotatably coupled to the base link to swing the support link around the second swinging axis. Moreover, the advancement and retraction drive means advances and retracts the tube with respect to the support link in the extending direction of the central axis of the tube.

According to the active trocar of an embodiment of the present invention, an instrument inserted through the tube, which is supported by the support link so that the tube can be advanced and retracted in the extending direction of the central axis, can be swung around the first swinging axis and the second swinging axis, which pass an intersection point located in the vicinity of the center of the hole opened in the abdominal wall of the body on the surgical bed and are orthogonal to each other. Moreover, the instrument can be advanced and retracted in the extending direction of the central axis of the tube. Therefore, axes of three degrees of freedom, which are two axes of swing and one axis of linear movement, come to intersect at a point in the vicinity of the center of the hole in the abdominal wall. Furthermore, since the first swing drive means and the second swing drive means can be arranged in the vicinity of the base and the advancement and retraction drive means can be arranged in the vicinity of the support link, these drive means can be concentratedly arranged in the vicinity of the abdominal wall. Accordingly, a small-sized surgery support robot with high rigidity can be provided, in which the occupied range of space within the operating room is greatly reduced with hardly reducing any degrees of freedom from the previously proposed one. Moreover, since the axes of three degrees of freedom, which are two axes of swing and one axis of linear movement, intersect at the point in the vicinity of the center of the hole in the abdominal wall, the position and the orientation of the instrument can be changed without any burden on the abdomen of the patient.

The active trocar of embodiments of the present invention may include a rotation drive means for rotating an instrument inserted through the tube around the central axis of the tube. With such a rotation drive means, a degree of freedom of rotation of the instrument around the central axis of the tube is added. Accordingly, a small-sized surgery support robot having four degrees of freedom equal to the conventional one can be provided, in which the occupied range of space within the operating room is greatly reduced. Moreover, the position and the orientation of the instrument can be changed with four degrees of freedom equal to the conventional one without any burden on the abdomen of the patient.
For a better understanding of the present invention, and to show how the same may be carried into effect, reference will now be made, by way of example to the accompanying drawings, in which:
Fig. 1 is a side view showing an example of an active trocar according to embodiments of the present invention;
Fig. 2 is a side view showing an example of a structure for fixing the active trocar of the above example to a surgical bed;
Fig. 3 is a side view showing constitutions of a first parallel crank quadric chain and a second parallel crank quadric chain of the active trocar of the above example;
Fig. 4 is an explanatory view showing motion of the active trocar of the above example;
Fig. 5 is an explanatory view showing motion of the active trocar of the above example;
Fig. 6 is an explanatory view showing motion of the active trocar of the above example;
Fig. 7 is an explanatory view showing a motion of a forceps in laparoscopic surgery;
Fig. 8 is an explanatory view showing a conventional method for driving the forceps; and
Fig. 9 is an explanatory view showing a conventional robot for driving the forceps.

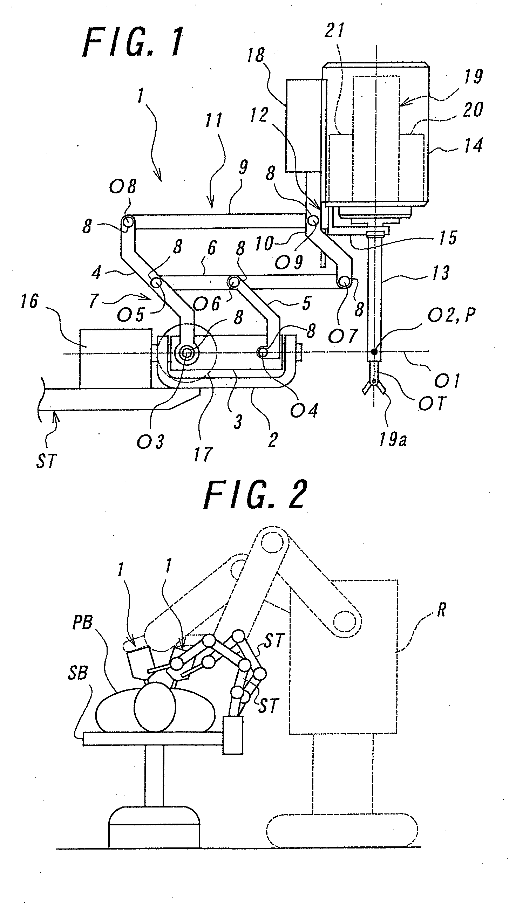

Next, detailed description will be made for an embodiment of the present invention by use of an example with reference to the drawings. Here, Fig. 1 is a side view showing an example of an active trocar according to an embodiment of the present invention.

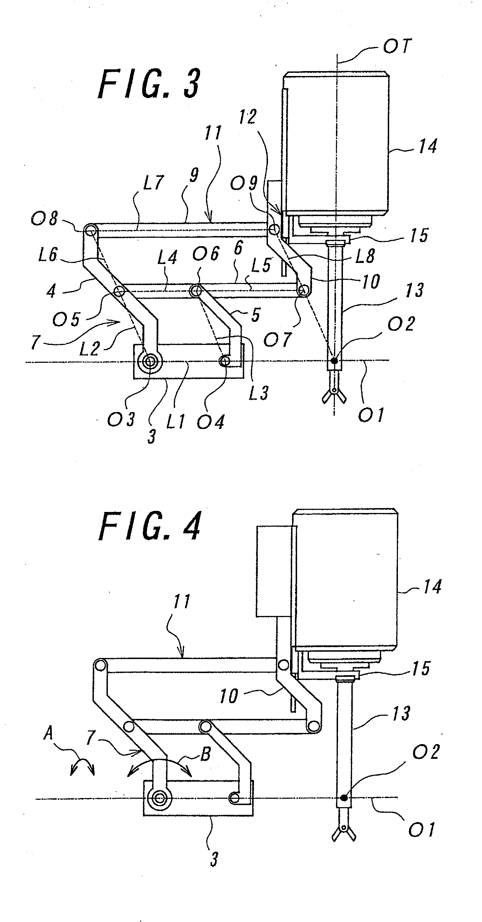

Fig. 2 is a side view showing an example of a structure for fixing the active trocar of the example to a surgical bed. Fig. 3 is a side view showing constitutions of a first parallel crank quadric chain and a second parallel crank quadric chain of the active trocar of the example. Reference numeral 1 in the drawings denotes the active trocar in the example.

The active trocar 1 of the example includes a base 2 and a first parallel crank quadric chain 7. Fig. 2 shows two active trocars of the example. The base 2 is supported by a typical articulated stand (passive positioner) ST, which is attached to a side of a surgical bed SB, to be fixed relatively to the surgical bed SB. The first parallel crank quadric chain 7 includes four links 3 to 6 containing a base link 3 supported on the base 2 so as to be able to swing around a first swinging axis O1 as shown in Fig. 1. The four links 3 to 6 are coupled to each other with pivots 8 so as to be able to rotate around rotating axes O3 to O6, which are parallel to each other and extended orthogonally to the first rotating axis O1, that is, in a direction orthogonal to the paper surface in Fig. 1. As shown in Fig. 3, lines L1, L2, L3 and L4 form a parallelogram. The line L1 connects the two rotating axes O3 and O4 of the base link 3. The line L2 connects the two rotating axes O3 and O5 of the link 4 coupled to the base link 3. The line L3 connects the two rotating axes O4 and O6 of the link 5 also coupled to the base link 3. The line L4 connects the two rotating axes O5 and O6 of the link 6 coupled to the links 4 and 5.

As shown in Fig. 1, the active trocar 1 of the example includes a second parallel crank quadric chain 11 including four links 4, 6, 9, and 10, containing two links which are respectively unitized with the link 4 of the first parallel crank quadric chain 7 rotatably connected to the base link 3 and the link 6 thereof where a line connecting two rotating axes thereof is parallel to the base link 3. For convenience, these two links are indicated by the reference numerals 4 and 6 similar to the links 4 and 6 of the first parallel crank quadric chain 7. The four links 4, 6, 9, and 10 are coupled to each other with the pivots 8 so as to be able to rotate individually around rotating axes O7 to O9 and O5, which are parallel to the rotating axes O3 to O6 of the first parallel crank quadric chain 7 and parallel to each other. As shown in Fig. 3, lines L5, L6, L7 and L8 form a parallelogram. The line L5 (part of the line L5 is overlapped with the line L4.) connects the two rotating axes O5 and O7 of the link 6. The line L6 connects the two rotating axes O5 and O8 of the link 4. The line L7 connects the two rotating axes O8 and O9 of the link 9 coupled to the link 4. The line L8 connects two rotating axes O7 and O9 of the support link 10 coupled to the links 6 and 9.

Here, as shown in Fig. 3, the support link 10 is arranged such that the line L8 connecting the two rotating axes O7 and O9 of the support link 10 intersects the line L1 connecting the two rotating axes O3 and O4 of the base link 3. The support link 10 is thus swung around a second swinging axis O2 by functions of the two parallel crank quadric chains 7 and 11 described above, wherein the second swinging axis O2 intersects the first swinging axis O1 at a position spaced from the base link 3. Moreover, through a typical linear guiding mechanism 12, the support link 10 supports an elevator 15, to which a tube 13 and an instrument case 14 are attached, in such an orientation that a central axis OT of the tube 13 passes an intersection point of the first swinging axis O1 and the second swinging axis O2 so as to be able to advance and retract the elevator in an extending direction of the central axis OT of the tube 13.

As shown in Fig. 1, the active trocar 1 of the example includes a typical geared motor 16 as a first swing drive means, a typical geared motor 17 as a second swing drive means, and a typical motor-drive linear movement mechanism 18 of a ball screw type as an advancement and retraction drive means. The geared motor 16 swings the base link 3 around the first swinging axis O1 with respect to the base 2 to swing the support link 10 around the first swinging axis O1. The geared motor 17 swings the link 4 of the first parallel crank quadric chain 7, the link 4 being rotatably coupled to the base link 3, with respect to the base link 3 to swing the support link 10 around the second swinging axis O2. The linear movement mechanism 18 advances and retracts the tube 13 with respect to the support link 10 in an extending direction of the central axis OT of the tube 13.

In addition, the active trocar 1 of the example, as shown in Fig. 1, includes an actuator 20 such as an electromagnetic solenoid and a typical geared motor 21 as a rotation drive means. The actuator 20 opens and closes a forceps tip 19a of an active forceps 19 accommodated in the instrument case 14, the forceps tip 19a being projected from a tip of the tube 13. The geared motor 21 rotates the forceps tip 19a around the central axis OT of the tube 13 to change the direction in which the forceps tip 19a is opened. The active forceps 19 itself is provided with a typical motor-drive linear movement mechanism of a ball screw type for tilting the forceps tip 19a with respect to the central axis OT of the tube 13.

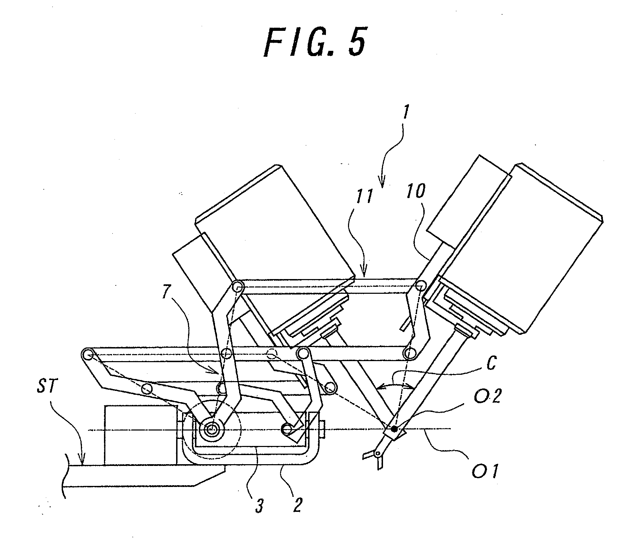

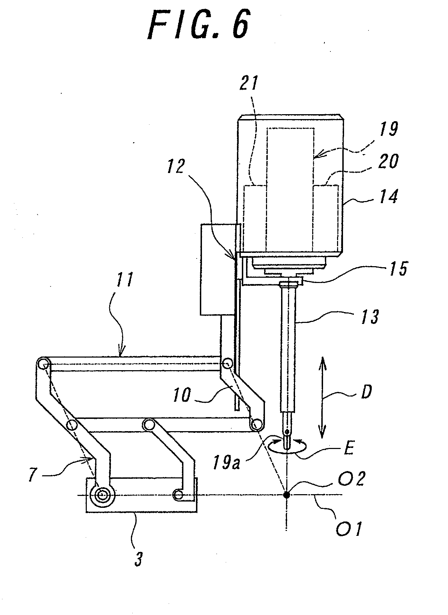

In the active trocar 1 according to the example, as shown in Fig. 2, the base 2 is fixed to the surgical bed SB by adjusting the articulated stand ST such that an intersection point P of the first swinging axis O1, the second swinging axis O2, and the central axis OT of the tube 13 is located in the vicinity of the center of the hole opened in the abdominal wall of the patient body PB laid on the surgical bed SB. When the forceps tip 19a of the forceps 19 is inserted through the tube 13, and the geared motors 16 and 17 and the motor-drive linear movement mechanism 18 of a ball screw type are properly operated, the geared motor 16 swings the base link 3 around the first swinging axis O1 with respect to the base 2 to swing the support link 10 around the first swinging axis O1 as shown by an arrow A in Fig. 4. The geared motor 17 swings the link 4 of the first parallel crank quadric chain 7 rotatably coupled to the base link 3 with respect to the base link 3 as shown by an arrow B in Fig. 4 to swing the support link 10 around the second swinging axis O2 as shown by an arrow C in Fig. 5. The motor-drive linear movement mechanism 18 of a ball screw type advances and retracts the tube 13 with respect to the support link 10 in the extending direction of the central axis OT of the tube 13 as shown by an arrow D in Fig. 6.

Furthermore, in the active trocar 1 of the example, the actuator 20 opens and closes the forceps tip 19a as shown in Figs. 5 and 6. The geared motor 21 rotates the forceps tip 19a around the central axis OT of the tube 13 as shown by an arrow E in Fig. 6. The active forceps 19 itself tilts the forceps tip 19a with respect to the central axis OT of the tube 13 by means of the motor-drive linear movement mechanism of a ball screw type thereof.

According to the active trocar 1 of the example, the forceps tip 19a inserted through the tube 13, which is supported by the support link 10 so that the tube can be advanced and retracted in the extending direction of the central axis OT, can be swung around the first swinging axis O1 and the second swinging axis O2, which pass the intersection point P located in the vicinity of the center of the hole opened in the abdominal wall of the patient body PB on the surgical bed SB and are orthogonal to each other. Moreover, the forceps tip 19a can be advanced and retracted in the extending direction of the central axis OT of the tube 13. Accordingly, axes of three degrees of freedom, which are two axes of swing and one axis of linear movement, come to intersect at the point P in the vicinity of the center of the hole in the abdominal wall. Furthermore, since the geared motors 16 and 17 can be arranged in the vicinity of the base 2 and the motor-drive linear movement mechanism 18 of a ball screw type can be arranged in the vicinity of the support link 10, the geared motors 16 and 17, and the motor-drive linear movement mechanism 18 can be concentratedly arranged in the vicinity of the abdominal wall. Therefore, as shown in Fig. 2, a small-sized surgery support robot with high rigidity can be provided, in which the occupied range of space within the operating room is greatly reduced compared with the conventional large-sized robot R with hardly reducing any degrees of freedom from that in the conventional one. Moreover, since the axes O1, O2, and OT of three degrees of freedom, which are two axes of swing and one axis of linear movement, intersect at the point P in the vicinity of the center of the hole in the abdominal wall as described above, the position and the orientation of the forceps tip 19a can be changed without any burden on the abdomen of the patient.

Furthermore, according to the active trocar 1 of the example, since the geared motor 21 for rotating the forceps 19a inserted through the tube 13 around the central axis OT of the tube 13 is provided, the degree of freedom of rotation of the forceps tip 19a around the central axis OT of the tube 13 is further added. Accordingly, a small-sized surgery support robot having four degrees of freedom equal to the conventional robot can be provided, in which the occupied range of space within the operating room is greatly reduced. Moreover, the position and the orientation of the forceps tip 19a can be changed with four degrees of freedom equal to the conventional robot without any burden on the abdomen of the patient.

According to the active trocar 1 of the example, the active trocar is provided with the actuator 20 for opening and closing the forceps tip 19a, and the active forceps 19 itself is provided with the typical motor-drive linear movement mechanism of a ball screw type for tilting the forceps tip 19a with respect to the central axis OT of the tube 3. Accordingly, an operation of the forceps can be performed by remote control as a master-slave robot.

Hereinbefore, description has been made based on the example shown in the drawings. However, the present invention is not intended to be limited to the above described example. For example, the second parallel crank quadric chain may be constituted in such a manner that the link 5 is extended and rotatably coupled to the link 9, and that the link 6 is rotatably coupled to only the links 5 and 10.
The shapes of the individual links may be properly changed according to need.

The articulated stand ST fixing the base 2 to the surgical bed SB may be supported by a support stood on a floor, not on the surgery bed. The instrument inserted in the tube 13 may be an endoscope or the like.

## Claims

1. An active trocar for swinging a tube inserted in a hole around a vicinity of a center of the hole and advancing and retracting the tube, the hole being opened in an abdominal wall of a body on a surgical bed, comprising:
a first parallel crank quadric chain, including four links containing a base link supported on a base to be swingable around a first swinging axis, the base being relatively fixed to the surgical bed, the four links being coupled to each other to be individually rotatable around rotating axes parallel to each other and orthogonal to the first swinging axis, lines each connecting the two rotating axes of the respective links forming a parallelogram;
a second parallel crank quadric chain, including four links containing two links respectively unitized with the link of said first parallel crank quadric chain rotatably coupled to said base link and with the link thereof in which a line connecting two rotating axes thereof is parallel to the line connecting the two rotating axes of said base link, the four links being coupled to each other to be individually rotatable around rotating axes parallel to each other and parallel to the rotating axes of said first parallel crank quadric chain, lines each connecting the two rotating axes of the respective links forming a parallelogram,
wherein a support link among the remainder other than said two links in the four links, in which a line connecting the two rotating axes thereof intersects the line connecting the two rotating axes of said base link, is swung around a second swinging axis intersecting the first swinging axis at a right angle at a position spaced from said base link, and
said support link supports the tube in such an orientation that a central axis of the tube passes an intersection point of the first swinging axis and the second swinging axis so as to be able to freely advance and retract the tube in an extending direction of the central axis of the tube;
a first swing drive means for swinging said base link around the first swinging axis with respect to the base to swing said support link around the first swinging axis;
a second swing drive means for swinging the link of said first parallel crank quadric chain rotatably coupled to said base link with respect to the base link to swing said support link around the second swinging axis; and
an advancement and retraction drive means for advancing and retracting the tube in the extending direction of the central axis of the tube with respect to said support link.

2. The active trocar according to claim 1, further comprising,
a rotation drive means for rotating an instrument inserted through the tube around the central axis of the tube.
